# EUROPEAN PATENT APPLICATION

(11) **EP 4 421 089 A1**
(43) Date of publication of application: **28.08.2024**
(21) Application number: 22883963.5
(22) Date of filing: 18.10.2022
(51) Int. Cl.: C07K 14/71, C07K 1/20, C07K 1/18

(54) **METHOD FOR PURIFYING FUSION PROTEIN HAVING IGG FC DOMAIN**

(30) Priority: 19.10.2021 KR 20210139445
(71) Applicant: Alteogen, Inc., Daejeon 34054 (KR)
(72) Inventor: PARK, Soon Jae, Daejeon 34054 (KR); BYUN, Minsoo, Daejeon 34054 (KR); NAM, Ki Seok, Daejeon 34054 (KR)
(74) Representative: Sagittarius IP
(86) International application number: PCT/KR2022/015837
(87) International publication number: WO 2023/068740

(57) **Abstract**

The present invention relates to: a method for purifying a fusion protein having an IgG Fc domain, comprising performing a multimodal chromatographic purification step on a raw material containing a fusion protein having an IgG Fc domain, so as to obtain a fusion protein having an IgG Fc domain with less than 0.05 EU/mg of endotoxins or a purity of size-exclusion-HPLC of 98% or higher; a method for purifying a fusion protein having an IgG Fc domain, the method not using anion exchange chromatography and comprising the steps of a) culturing cells for producing a fusion protein having an IgG Fc domain, b) recovering the protein from the cells cultured in step a), c) purifying the protein recovered in step b) through primary chromatography and d) purifying, through multimodal chromatography and cation exchange chromatography, the protein purified by primary chromatography in step c); and a composition comprising the fusion protein having an IgG Fc domain, purified by the method.

## Description

### [Technical Field]

The present invention relates to: a method for purifying a fusion protein having an IgG Fc domain, wherein a raw material containing a fusion protein having an IgG Fc domain is subjected to a multimodal chromatography purification step to obtain a fusion protein having an IgG Fc domain with endotoxin < 0.05 EU/mg or a size exclusion HPLC purity of 98% or more; a method for purifying a fusion protein having an IgG Fc domain, the method including a) culturing cells producing a fusion protein having an IgG Fc domain, b) recovering a protein from the cells cultured in step a), c) purifying the protein, recovered in step b), by primary chromatography, d) purifying the protein, purified by primary chromatography in step c), by multimodal chromatography and cation exchange chromatography, wherein no anion exchange chromatography is used; a fusion protein having an IgG Fc domain purified by the method; and a composition containing the fusion protein.

### [Background Art]

Human immunoglobulin G (IgG) Fc domain is a factor that shows persistence in protein medicines through the fusion with a domain exhibiting the function of a protein. Therefore, many commercially available protein medicines have been developed in the form of an Fc fusion protein.

Vascular endothelial growth factor (VEGF) is an important factor that increases angiogenesis and vascular permeability. Especially, VEGF is overexpressed in tumors to abnormally promote angiogenesis and tumor proliferation (Oncogene (2004) 23, 1745-1753). It has been also reported that abnormal angiogenesis is importantly involved in other diseases in addition to tumorigenesis. Abnormal angiogenesis in VEGF-related mechanisms is associated with wet macular degeneration, diabetic retinopathy, retinal vein occlusion macular edema, and the like, which are ophthalmic diseases (J. Korean Med. Assoc. (2014), 57(7), 614-623).

For the treatment of ophthalmic diseases, pegaptanib (RNA aptamer), ranibizumab (monoclonal IgG antibody fragment (Fab)), and bevacizumab (monoclonal IgG antibody) are used, and aflibercept (VEGFR1 and VEGFR2 fused with IgG1 Fc) was approved as a medicine for wet macular degeneration in the USA in 2011 (Biol. Ther. (2012) 2(3) 1-22, Drug Design, Development and Therapy (2013) 3(7), 711-722).

### [Disclosure]

### [Technical Problem]

The present inventors have developed an effective method for purifying a fusion protein having an IgG Fc domain by performing a multimodal chromatography purification step.

### [Technical Solution]

In accordance with an aspect of the present invention, the present invention provides a method for purifying a fusion protein having an IgG Fc domain, wherein a raw material containing a fusion protein having an IgG Fc domain is subjected to a multimodal chromatography purification step to obtain a fusion protein having an IgG Fc domain with endotoxin < 0.05 EU/mg or a size exclusion HPLC purity of 98% or more.

In accordance with another aspect of the present invention, the present invention provides a method for purifying a fusion protein having an IgG Fc domain, the method including: a) culturing cells producing a fusion protein having an IgG Fc domain; b) recovering a protein from the cells cultured in step a); c) purifying the protein, recovered in step b), by primary chromatography; d) purifying the protein, purified by primary chromatography in step c), by multimodal chromatography and cation exchange chromatography, wherein no anion exchange chromatography is used.

In accordance with still another aspect of the present invention, the present invention provides a fusion protein having an IgG Fc domain purified by the method and a composition containing the same.

### [Advantageous Effects]

The use of the purification method of the present invention can provide an efficient mass production method and supply of a fusion protein having an IgG Fc domain.

### [Brief Description of Drawings]

FIG. 1 shows a chromatogram obtained by subjecting a fusion protein having an IgG Fc domain, especially, aflibercept, obtained from CHO cell culture, to Protein A chromatography in a binding and elution mode by using MabSelect SuRe resin.
FIG. 2 shows a chromatogram obtained by subjecting the fusion protein having an IgG Fc domain, especially, aflibercept, obtained from CHO cell culture, to multimodal chromatography in a binding and elution mode by using Capto Adhere resin after the purification step (Protein A chromatography) in FIG. 1.
FIG. 3 shows a chromatogram obtained by subjecting the fusion protein having an IgG Fc domain, especially, aflibercept, obtained from CHO cell culture, to cation exchange chromatography in a binding and elution mode by using Capto S resin after the purification steps (Protein A affinity chromatography and multimodal chromatography) in FIGS. 1 and 2.
FIG. 4 shows the isoelectric focusing analysis results of fractions purified and eluted in the purification step (Capto S cation exchange chromatography) in FIG. 3. Lane 1 represents internal reference 1, and Lane 2 represents internal reference 2. Lane 3 represents the analysis results of a sample collected with a cation exchange chromatography (CEX) elution volume of 3 CV, and Lanes 4, 5, 6, 7, and 8 represent the analysis results of samples collected with a CEX elution volume increasing by 0.5 CV per each lane.
FIG. 5 shows a chromatogram obtained by subjecting the fusion protein having an IgG Fc domain, especially, aflibercept, obtained from CHO cell culture, to cation exchange chromatography in a binding and elution mode by using SP-550C resin after the purification steps (Protein A affinity chromatography and multimodal chromatography) in FIGS. 1 and 2.
FIG. 6 shows the isoelectric focusing analysis results of fractions purified and eluted in the purification step (SP-550C cation exchange chromatography) in FIG. 5. Lane 1 represents the analysis results of a sample collected with a CEX elution volume of 4 CV, and Lanes 2 and 3 represent the analysis results of samples collected at a CEX elution volume of 8 CV and 10 CV, respectively.
FIG. 7 shows a chromatogram of salt gradient elution obtained by cation exchange chromatography.
FIG. 8 shows the isoelectric focusing analysis results by fractions of salt gradient elution obtained by cation exchange chromatography. In the salt gradient elution, the yield of the target protein was reduced due to isoform removal.
FIG. 9 shows a chromatogram obtained by subjecting the fusion protein having an IgG Fc domain, especially, aflibercept, obtained by CHO cell culture, to anion chromatography in a flow-through mode using Capto Q for a comparative evaluation with Capto Adhere resin multimodal chromatography.
FIG. 10 shows a chromatogram obtained by subjecting the fusion protein having an IgG Fc domain, especially, aflibercept, obtained by CHO cell culture, to multimodal chromatography in a binding and elution mode using Capto Adhere resin in order to conduct a comparative evaluation with Capto Q resin anion exchange chromatography.
FIG. 11 shows the host cell protein (HCP) analysis results on the same samples after purification for a comparative evaluation between anion chromatography and multimodal chromatography.
FIG. 12 shows the oxidation analysis results of methionine residues of the purified aflibercept of the present invention and commercially available aflibercept.
FIG. 13 shows the oxidation analysis results of histidine residues of the purified aflibercept of the present invention and commercially available aflibercept.
FIG. 14 shows the deamidation analysis results of the respective amino acid residues of the purified aflibercept of the present invention and commercially available aflibercept.
FIG. 15 shows the dehydration analysis results of the respective amino acid residues of the purified aflibercept of the present invention and commercially available aflibercept.

### [Best Mode for Carrying Out the Invention]

Hereinafter, the present invention will be described in detail. Each description and embodiment disclosed in the present invention may also be applied to other descriptions and embodiments containing the common matters. Additionally, all combinations of various elements disclosed in the present invention fall within the scope of the present invention. Furthermore, the documents described in the present invention can be incorporated herein by reference. Furthermore, the scope of the present invention is not limited by the specific description below.

An aspect of the present invention provides a method for purifying a fusion protein having an IgG Fc domain, wherein a raw material containing a fusion protein having an IgG Fc domain is subjected to a multimodal chromatography purification step to obtain a fusion protein having an IgG Fc domain with endotoxin < 0.05 EU/mg or a size exclusion HPLC purity of 98% or more.

The term "fusion protein having an IgG Fc domain" used herein refers to a fusion protein containing an IgG Fc domain. The raw material containing a fusion protein having an IgG Fc domain may be produced from cell culture using an animal cell line, but is not limited thereto.

In an embodiment, the fusion protein of the present invention may be a vascular endothelial growth factor (VEGF) antagonist, but is not limited thereto.

In an embodiment, the fusion protein of the present invention may contain an IgG Fc domain and VEGF receptor-1 and -2, but is not limited thereto.

In an embodiment, the VEGF receptor-1 and -2 may be extracellular domains of VEGF receptor-1 and -2, but are not limited thereto.

In an embodiment, the VEGF receptors may be human VEGF receptors, but are not limited thereto.

In an embodiment, the fusion protein of the present invention may be aflibercept, but is not limited thereto.

The term "IgG Fc" used herein refers to an Fc region, which is a constant region of an immunoglobulin IgG. It would be obvious that any protein having an amino acid sequence with deletion, modification, substitution, conservative substitution, or addition of some amino acids can be used in the present invention as long as the protein has activity identical or equivalent to that of the Fc region of the immunoglobulin IgG.

The immunoglobulin IgG Fc may consist of CH1, CH2, CH3, and CH4 domains of IgG and may include a hinge region.

In addition, the immunoglobulin IgG Fc region of the present invention includes an amino acid sequence of mutant as well as a sequence of native immunoglobulin IgG Fc region. The amino acid sequence mutant means a mutant having a different sequence from a native amino acid sequence due to deletion, insertion, non-conservative or conservative substitution, or a combination thereof of at least one amino acid residue in the native amino acid sequence.

In an embodiment, the IgG Fc of the present invention may be human IgG Fc.

In an embodiment, the IgG Fc of the present invention may be an Fc domain of IgG1, IgG2, IgG3, or IgG4, and specifically may be an Fc domain of IgG1, but is not limited thereto.

In the invention, the "raw material containing a fusion protein having an IgG Fc domain" may include unpurified cells, a culture thereof, a lysate thereof obtained through cell culture or the like and may include a fusion protein having an IgG Fc domain, but is not limited thereto.

The method for purifying the fusion protein having an IgG Fc domain includes a multimodal chromatography purification step.

The term "chromatography" used hereinafter refers to any process, in which components of a mixture are separated by passing the mixture through a medium such that the components of the mixture pass through the medium at different rates. Examples of the chromatography may include column chromatography, planar chromatography, thin layer chromatography, gas chromatography, liquid chromatography, fast protein liquid chromatography (FPLC), high-performance liquid chromatography (HPLC), ion exchange chromatography (cation exchange chromatography, anion exchange chromatography), size exclusion chromatography, multimodal chromatography, hydrophobic interaction chromatography, and the like.

In each step of the purification process of the present invention, exemplary chromatography processes or apparatuses are described, and these can be applied to all types of chromatography described above.

The chromatography in each step of the present invention may include a sample injection stage, an equilibration stage, a washing stage, and an elution stage. Specifically, the respective stages may be performed in a changed order, and an additional stage (e.g., an equilibration stage or a washing stage) may be added before, after, or between the stages, and one stage may include another stage. For example, an equilibration stage may be further added before the sample injection stage, and the washing stage may be included in the equilibration stage, or the equilibration stage may be included in the washing stage. The equilibration stage of the present invention may be a stage of equilibrating a resin by passing an equilibration buffer through a column; the washing stage of the present invention may be a stage of washing the column with a buffer to remove impurities adsorbed on the resin; and the elution stage may be a stage of eluting and/or recovering a target protein.

As one example, the buffers in the chromatography of the present invention may contain at least one selected from the group consisting of tromethamine, sodium phosphate, sodium acetate, citric acid, and acetic acid, but are not limited thereto. The buffers may be used in the equilibration stage, washing stage, and elution stage.

In an embodiment, the elution stage of the present invention may use a salt gradient, a pH gradient, or a combination thereof.

In an embodiment, the elution stage of the present invention may be performed in a binding and elution mode.

In an embodiment, the elution stage of the present invention may not use a flow-through mode.

The term "flow-through" used herein refers to a mode in which a flow through is collected by chromatography, and the term "binding and elution" used herein refers to a mode in which a target protein is bound to a ligand attached to a resin through the mixed-mode interaction and releases molecules from the resin using the composition of a buffer composition, pH or etc. The flow-through mode may be a mode in which only (a) substance(s) bound to the column resin is(are) removed under particular conditions, and the binding and elution mode may be a mode in which (a) substance(s) unbound to the column resin is(are) removed under particular conditions and other substance(s) unbound to the column resin is(are) recovered.

In the purification of an antibody and fusion protein having an IgG Fc domain of the present invention, the fusion protein is purified by multimode chromatography with a binding and elution mode, instead of adopting a generally used flow-through mode in which a flow through is collected by anion exchange chromatography or multimodal chromatography, thereby effectively removing host cell proteins, endotoxins, viruses, protein A leachates, and the like compared with a flow-through mode using anion exchange chromatography and/or multimode chromatography. Furthermore, the use of the purification method of the present invention enables the production of a high-purity fusion protein only through a simple three-step chromatography purification process and enables effective purification of a fusion protein having an IgG Fc domain with an isoelectric point in the range of pH 6-8, especially aflibercept.

The term "multimodal chromatography" used herein refers to a chromatography that utilizes at least one type of interaction between a stationary phase and (a) target protein(s) in order to separate (a) target protein(s). Examples of the interaction may include specific interactions for protein size, charge, hydrophobic, and/or hydrophilic properties. The multimodal chromatography may be used interchangeably with mixed mode chromatography. The multimodal chromatography of the present invention includes physical multimodal chromatography and chemical multimodal chromatography.

The multimodal chromatography of the present invention may include an equilibration stage, a sample injection stage, a washing stage, and an elution stage.

In an embodiment, the buffers used in each stage of the multimodal chromatography of the present invention may contain at least one selected from the group consisting of tromethamine, sodium phosphate, and sodium acetate, but are not limited thereto.

In an embodiment, the equilibration stage of the multimodal chromatography of the present invention may include passing 1 to 10 column volume (CV), 2 to 8 CV, 4 to 6 CV, or 5 CV of a buffer through the column at a flow rate of 100 to 200 cm/hr, 110 to 190 cm/hr, 120 to 180 cm/hr, 130 to 170 cm/hr, 140 to 160 cm/hr, or 150 cm/hr, but is not limited thereto.

In an embodiment, the equilibration stage of the multimodal chromatography of the present invention may be performed at about pH 6-8, specifically pH 7, but is not limited thereto.

In an embodiment, the buffer used in the equilibration stage of the multimodal chromatography of the present invention may have about pH 6-8, specifically pH 7, but is not limited thereto.

In an embodiment, the buffer used in the equilibration stage of the multimodal chromatography of the present invention may contain tromethamine, sodium phosphate, or a combination thereof, but is not limited thereto.

In an embodiment, in the washing stage of the multimodal chromatography of the present invention, 1 to 10 column volume (CV), 2 to 8 CV, 4 to 6 CV, or 5 CV of a buffer may be passed through the column, but is not limited thereto.

In an embodiment, in the elution stage of the multimodal chromatography of the present invention, 1 to 10 column volume (CV), 3 to 9 CV, 5 to 8 CV, or 7 CV of a buffer may be passed through the column, but is not limited thereto.

In an embodiment, the elution stage of the multimodal chromatography of the present invention may be performed at about pH 3-7, about pH 4-6, and specifically about pH 5, but is not limited thereto.

In an embodiment, the buffer used in the elution stage of the multimodal chromatography of the present invention may have about pH 3-7, about pH 4-6, and specifically about pH 5, but is not limited thereto.

The term "about" used herein may be presented before a specific numerical value. The term "about" used herein encompasses not only the exact number recited after the term, but also a range that is close to or nearly equivalent to that number. Considering the context in which the number is presented, it can be determined whether any number is close to or nearly equivalent to the particular number recited. As an example, the term "about" may refer to a range of -10% to +10% of a numerical value. As another example, the term "about" may refer to a range of -5% to +5% of a given numerical value. However, the term is not limited thereto.

In an embodiment, the buffer used in the elution stage of the multimodal chromatography of the present invention may contain sodium phosphate, sodium acetate, or a combination thereof, but is not limited thereto.

In an embodiment, the elution stage of the multimodal chromatography of the present invention may use a salt gradient, a pH gradient, or a combination thereof, specifically, a pH gradient.

In an embodiment, the elution stage of the multimodal chromatography of the present invention may be performed in a binding and elution mode.

In an embodiment, the elution stage of the multimodal chromatography of the present invention may not use a flow-through mode.

As an example, the resin used in the multimodal chromatography of the present invention may be at least one selected from the group consisting of Capto adhere^{™}, Capto MMC^{™}, Capto MMC ImpRes^{™}, Capto adhere ImpRes^{™}, Nuvia aPrime 4A, Capto Core 400^{™}, and Capto Core 700^{™}, and the multimodal ligand may be specifically Capto adhere and, more specifically, N-benzyl-N-methylethanolamine, but is not limited thereto.

In an embodiment, the multimodal ligand used in the multimodal chromatography of the present invention may include a material having multimodal functions of ionic binding, hydrogen binding, and hydrophobic binding.

In an embodiment of the present invention, the resin was equilibrated by passing 5 CV of an equilibration buffer through the column at a flow rate of 150 cm/hr. As the equilibration buffer, a tromethamine and/or sodium phosphate buffer of about pH 7 was used. Thereafter, the obtained depth filtration recovery solution was injected, and the column was washed with 5 CV of an equilibration buffer. A target protein was eluted and recovered by flowing 7 CV of an elution buffer through the column. As the elution buffer, a sodium phosphate and/or sodium acetate buffer of about pH 5 was used (Example 5).

The purification method of the present invention may further include a cation exchange chromatography purification step.

In an embodiment, the purification method of the present invention may include a cation exchange chromatography purification step before, after, or simultaneously with the multimodal chromatography purification step. Specifically, the purification method of the present invention may include a cation exchange chromatography purification step after the multimodal chromatography purification step, but is not limited thereto.

As used herein, the term "ion exchange chromatography" refers to the resin exchange using the reversible interaction of the net charges between the medium and the protein surfaces, that is, the difference in ionic bond strength. The cation exchange chromatography used in the present invention means ion chromatography using a column filled with a cation exchange resin, and from the above step, cation exchange chromatography may be performed to additionally remove impurities and selectively separate an isomer with a desired isoelectric point.

The cation exchange chromatography of the present invention may include an equilibration stage, a sample injection stage, a first washing stage, a second washing stage, and an elution stage.

In an embodiment, buffers used in each stage of the cation exchange chromatography of the present invention may contain sodium phosphate, sodium acetate, or a combination thereof, but are not limited thereto.

In an embodiment, the equilibration stage of the cation exchange chromatography of the present invention may include passing 5 to 15 CV, 7 to 13 CV, 8 to 12 CV, 9 to 11 CV, or 10 CV of a buffer through the column at a flow rate of 100 to 200 cm/hr, 110 to 190 cm/hr, 120 to 180 cm/hr, 130 to 170 cm/hr, 140 to 170 cm/hr, or 160 cm/hr, but is not limited thereto.

In an embodiment, the equilibration stage of the cation exchange chromatography of the present invention may be performed at about pH 3-7, about pH 4-6, and specifically pH 5, but is not limited thereto.

In an embodiment, the buffer used in the equilibration stage of the cation exchange chromatography of the present invention may have about pH 3-7, about pH 4-6, and specifically pH 5, but is not limited thereto.

In an embodiment, in the first washing stage of the cation exchange chromatography of the present invention, 1 to 10 column volume (CV), 2 to 8 CV, 4 to 6 CV, or 5 CV of a buffer may be passed through the column, but is not limited thereto.

In an embodiment, the second washing stage of the cation exchange chromatography of the present invention may be for removing highly acidic fractions among the target protein related substances and may be performed by passing 1 to 5 CV, 2 to 4 CV, or 3 CV of a buffer through the column at about pH 3-7, about pH 4-6, about pH 5-6, specifically about pH 5.9, but is not limited thereto.

In an embodiment, in the elution stage of the cation exchange chromatography of the present invention, 5 to 15 CV, 8 to 12 CV, or 10 CV of a buffer may be passed through the column, but is not limited thereto.

In an embodiment, the elution stage of the cation exchange chromatography of the present invention may be performed at about pH 3-8, pH 6-8, about pH 4-7, pH 5-7, pH 6-7, and specifically about pH 6.5, but is not limited thereto.

In an embodiment, the buffer used in the elution stage of the cation exchange chromatography of the present invention may have about pH 3-8, pH 6-8, about pH 4-7, pH 5-7, pH 6-7, and specifically about pH 6.5, but is not limited thereto.

In an embodiment, the elution stage of the cation exchange chromatography of the present invention may use a salt gradient, a pH gradient, or a combination thereof, specifically, a salt gradient.

In an embodiment, the elution stage of the cation exchange chromatography of the present invention may be performed in a binding and elution mode.

In an embodiment, the elution stage of the cation exchange chromatography of the present invention may not use a flow-through mode.

In an example of the present invention, the resin was equilibrated by passing 10 CV of an equilibration buffer at a rate of 160 cm/hr. As the equilibration buffer, a buffer of about pH 5 may be used and, preferably, a sodium phosphate and/or sodium acetate buffer may be used. The recovery fluid obtained by the multimodal chromatography step was injected, and the column was washed with 5 CV of an equilibration buffer. To remove highly acidic fractions among the target protein related substances, washing was performed by passing 3 CV of a sodium phosphate and/or sodium acetate buffer of about pH 5.9 through the column. As the elution buffer, 10 CV of a sodium phosphate and/or sodium acetate buffer of about pH 6.5 was used, thereby recovering a target protein (Example 6). The present inventors have developed a method capable of effectively purifying a fusion protein with an isoelectric point in the range of pH 6-8 by using a cation exchange resin, after the multimodal chromatography binding and elution mode, to further remove residual host cell proteins, endotoxin, aflibercept related substances, and the like.

Examples of the resin usable in the cation exchange chromatography of the present invention may include CM Sepharose Fast Flow, Capto ImpRes SP, SOURCE 30 S, SOURCE 15 S, Eshmuno S, Eshmuno CPX, Eshmuno CP-FT, Eshmuno CPS, Fractogel EMD SO3-, Fractogel EMD SE, Capto MMC, Macro-Prep 25 S, Macro-Prep CM, UNOsphere S, Macro-Prep High S, POROS^{™} XS, POROS^{™} 50 HS, Nuvia S Resin, Nuvia HR-S, Nuvia cPrim, Exhmuno CMX^{™}, Capto MMC ImpRes^{™}, Nuvia cPrime, TOYOPEARL SP-650,TOYOPEARL GigaCap S-650, TOYOPEARL CM-650, TOYOPEARL GigaCap CM-650, TOYOPEARL Salfate-650, TOYOPEARL MegaCap II SP-550, and TOYOPEARL SP-550, but is not limited thereto. Preferably, the resin used in the cation exchange chromatography of the present invention may be at least one selected from the group consisting of Capto S^{™}, Capto ImpRes SP, and Eshmuno CPS, but is not limited thereto.

In an embodiment, the ligand of the cation exchange chromatography may include at least one of sulfonic acid (-SOs), sulfopropyl, and carboxymethyl, and specifically, sulfonic acid.

The cation exchange resin of the cation exchange chromatography of the present invention refers to a synthetic resin that is added to another aqueous solution to serve to exchange its own cations with specific cations in the aqueous solution, and the cation exchange column can adsorb a protein with positive ions above its own isoelectric point.

In one embodiment, the functional group of the cation exchange chromatography resin may be one selected from the group consisting of methylsulfonate (S), sulfopropyl (SP), carboxymethyl (CM), polyaspartic acid, sulfoethyl (SE), and sulfopropyl (SP), phosphate (P), and sulfonate (S).

In the purification method of the present invention, size exclusion chromatography, hydrophobic interaction chromatography, and anion exchange chromatography may not be performed, but is not limited thereto. In the present invention, size exclusion chromatography, hydrophobic interaction chromatography, and anion exchange chromatography is not performed to facilitate scale-up according to the sample concentration process and purification scale, prevent a hydrophobic portion of the protein structure from being exposed to a salt, and increase the level of purification.

Even in such a case, the size exclusion chromatography, hydrophobic interaction chromatography, and anion exchange chromatography may be used for purposes (e.g., investigation or verification of fusion protein purity) other than the purification of a fusion protein having an IgG Fc domain.

In an embodiment, the purification method of the present invention may not use anion exchange chromatography, which is generally performed for the purification of antibodies and fusion proteins having IgG Fc domains.

The anion exchange chromatography of the present invention refers to ion chromatography using a column filled with an anion exchange resin.

The method for purifying a fusion protein having an IgG Fc domain of the present invention may further include a filtration step before or after each step, and the filtration step may be any one selected from the group consisting of depth filtration, ultrafiltration, and diafiltration, but is not limited thereto. The filtration may be performed in one or more runs.

The term "depth filtration" used herein refers to filtration that uses a filter agent with a large particle diameter and a small coefficient of uniformity to internally capture flocs that have passed through the surface of the filter layer without being filtered out.

The term "ultrafiltration" used herein refers to a process that can fractionate substances dissolved or dispersed in a liquid according to the particle size or molecular weight by using pressure and a membrane.

The term "diafiltration" used herein refers to a process that only some solutes are removed from a fluid containing a solvent and two or more solutes with different molecular sizes by using dialysis.

In accordance with another aspect of the present invention, there is provided a method for purifying a fusion protein having an IgG Fc domain, the method including: a) culturing cells producing a fusion protein having an IgG Fc domain; b) recovering a protein from the cells cultured in step a); c) purifying the protein, recovered in step b), by primary chromatography; d) purifying the protein, purified by primary chromatography in step c), by multimodal chromatography and cation exchange chromatography, wherein no anion exchange chromatography is used. In step d), the multimodal chromatography may be performed before, after, or a combination of before and after the cation exchange chromatography.

The fusion protein having an IgG Fc domain, finally purified by the method for purifying a fusion protein having an IgG Fc domain, may have a size exclusion HPLC purity of 98% or more, endotoxin < 0.05 EU/mg, or a pl of 6.0-8.0.

Through the purification method of the present invention, the present inventors can produce a high-purity fusion protein by only a simple three-step chromatography purification process without using size exclusion chromatography, hydrophobic interaction chromatography, anion exchange chromatography, and the like. Furthermore, a fusion protein having an IgG Fc domain having an isoelectric point in the range of pH 6-8, especially, aflibercept, can be effectively purified.

The fusion protein having an IgG Fc domain, multimodal chromatography, cation exchange chromatography, and anion exchange chromatography are as described in other embodiments.

In the step of a) culturing cells producing a fusion protein having an IgG Fc domain in the present invention, the cells may be animal cells, specifically, hamster ovary cells (CHO cells), but are not limited thereto.

The term "culturing" used herein refers to growing the cells in an appropriately controlled environmental condition. The culturing of the present application may be performed according to appropriate media or culturing conditions known in the art. Such culturing may be easily controlled by a person skilled in the art according to the selected type of cells.

In step b) of recovering a protein from the cells cultured in step a) in the present invention, the recovering may be collecting a fusion protein having a desired IgG Fc domain by using a suitable method known in the art according to the culturing method of the present invention. For example, centrifugation, filtration, depth filtration, extraction, ultrafiltration, dialysis, and the like may be used in combination, and the fusion protein having a desired IgG Fc domain can be recovered from media or cells by using a suitable method known in the art.

In step c) of purifying the protein, recovered in step b), by primary chromatography in the present invention, the primary chromatography may be affinity chromatography, specifically, Protein A affinity chromatography, but is not limited thereto.

The term "affinity chromatography" used herein refers to chromatography using a high-specificity interaction between a target molecule and a ligand.

The affinity chromatography of the present invention may include an equilibration stage, a sample injection stage, a first washing stage, a second washing stage, and an elution stage.

In an embodiment, the buffers used in each stage of the affinity chromatography of the present invention may contain at least one selected from the group consisting of tromethamine, sodium phosphate, sodium acetate, and citric acid, but are not limited thereto.

In an embodiment, the equilibration stage of the affinity chromatography of the present invention may include passing 2 to 8, 4 to 6 CV, or 5 CV of a buffer through a column, but is not limited thereto.

In an embodiment, the equilibration stage of the affinity chromatography of the present invention may be performed at about pH 6-8, specifically pH 7, but is not limited thereto.

In an embodiment, the buffer used in the equilibration stage of the affinity chromatography of the present invention may have about pH 6-8, specifically pH 7, but is not limited thereto.

In an embodiment, the buffer used in the equilibration stage of the affinity chromatography of the present invention may contain tromethamine, sodium phosphate, or a combination thereof, but is not limited thereto.

In an embodiment, the buffer used in the first washing stage of the affinity chromatography of the present invention may contain citric acid, acetic acid, or a combination thereof, and may further include L-arginine and/or urea, but is not limited thereto.

In an embodiment, the first washing stage of the affinity chromatography of the present invention may be performed at about pH 3-7, about pH 4-7, about pH 5-6.5, specifically, about pH 5.9, but is not limited thereto.

In an embodiment, the first washing stage of the affinity chromatography of the present invention may be passing 5 to 15 CV, 7 to 13 CV, 8 to 12 CV, 9 to 11 CV, or 10 CV of a buffer through a column at about pH 3-7, about pH 4-7, pH 5-6.5, specifically, about pH 5.9, but is not limited thereto.

In an embodiment, the buffer used in the second washing stage of the affinity chromatography of the present invention may contain citric acid, acetic acid, or a combination thereof, but is not limited thereto.

In an embodiment, the second washing stage of the affinity chromatography of the present invention may be performed at about pH 3-7, about pH 4-6, pH 5-6, specifically, about pH 5.6, but is not limited thereto.

In an embodiment, the second washing stage of the affinity chromatography of the present invention may be passing 1 to 7 CV or 3 to 5 CV of a buffer through a column at about pH 3-7, about pH 4-6, pH 5-6, specifically, pH 5.6, but is not limited thereto.

In an embodiment, the elution stage of the affinity chromatography of the present invention may be performed at about pH 1-5, about pH 2-4, about pH 3-4, specifically, about pH 3.5, but is not limited thereto.

In an embodiment, the elution stage of the affinity chromatography of the present invention may be passing 1 to 7 CV or 3 to 5 CV of a buffer through a column at about pH 1-5, about pH 2-4, about pH 3-4, specifically, about pH 3.5, but is not limited thereto.

In an embodiment, the buffer used in the elution stage of the affinity chromatography of the present invention may contain citric acid, acetic acid, or a combination thereof, but is not limited thereto.

In an embodiment, the elution stage of the affinity chromatography of the present invention may use a salt gradient, a pH gradient, or a combination thereof, specifically, a pH gradient.

In an embodiment, the elution stage of the affinity chromatography of the present invention may be performed in a binding and elution mode.

In an embodiment, the elution stage of the affinity chromatography of the present invention may not use a flow-through mode.

For example, the column usable in the affinity chromatography of the present invention may be MabSelect PrismA, MabSelect, MabSelect Xtra, MabSelect SuRe, MabSelect SuRe LX, MabSelect SuRe pcc, or MabCaptureC^{™}.

For example, the resin used in the affinity chromatography of the present invention may be at least one selected from the group consisting of MabSelect SuRe resins having alkali-stabilized Protein A-derived ligands, but is not limited thereto.

In an embodiment of the present invention, about 5 column volumes (CV) of a tromethamine and/or sodium phosphate buffer of pH 6 to 8, preferably about pH 7, are passed through the column at a flow rate with a retention time of 7.5 minutes to achieve resin equilibration. Thereafter, the harvested cell culture fluid obtained in Example 1 was injected into the equilibrated column, and the column was washed with 3 CV of an equilibration buffer to remove impurities adsorbed on the resin. Thereafter, 10 CV of washing buffer 1 and 3-5 CV of washing buffer 2 were sequentially passed through the column to further remove impurities. A citric acid and/or acetic acid buffer of pH 5-6.5, preferably about pH 5.9, was used as washing buffer 1, wherein washing buffer 1 may further contain L-arginine and/or urea. A citric acid and/or acetic acid buffer of about pH 5.6 was used as washing buffer 2. Thereafter, 5 CV of a citric acid and/or acetic acid buffer of about pH 3.5 as an elution solution was passed through the column to recover a target protein (Example 2).

The step d) of purifying the protein, purified by primary chromatography in step c), by multimodal chromatography and cation exchange chromatography is as described in other aspects.

In an embodiment, the purifying by the multimodal chromatography in step d) may include: (a) loading the protein, purified by primary chromatography, onto a multimodal chromatography column equilibrated with a buffer of pH 6-8; and (b) injecting a buffer having a pH lower than that in step (a) into the chromatography column to elute and harvest the purified protein under conditions of pH 4-6, but is not limited thereto.

In step d), the purifying by the cation exchange chromatography is as described in other aspects.

In an embodiment, the purifying by the cation exchange chromatography may include: (a) loading the protein, purified by the preceding chromatography, onto a cation exchange chromatography column equilibrated with a buffer of pH 4-6; and (b) harvesting a fusion protein having an IgG Fc domain with a pl of 6.0-8.0 by elution with a buffer having a pH higher than that of the buffer in step (a), but is not limited thereto.

In an embodiment, in step (b), the buffer having a higher pH than the buffer in step (a) may be about pH 5-9, about pH 6-9, or pH 5-8, and specifically pH 6-8, but is not limited thereto.

In an embodiment, at least one of a depth filtration step, an ultrafiltration step, a dialysis filtration step, and virus inactivation step is additionally performed before, after, or a combination of before and after at least one of steps c) and d), but is not limited thereto.

In an embodiment, at least one step selected from the group consisting of the purifying by primary chromatography of step c) and the purifying by multimodal chromatography and cation exchange chromatography of step d) includes an elution stage, and the elution stage may use a salt gradient, a pH gradient, or a combination thereof, specifically a pH gradient, but is not limited thereto.

Still another aspect of the present invention provides a composition containing a fusion protein having an IgG Fc domain, purified by the method for purifying a fusion protein having an IgG Fc domain of the present invention.

The fusion protein having an IgG Fc domain, purification method therefor, and the like are as described in other aspects.

The composition of the present invention may contain a Peak 1 fraction. The Peak 1 fraction may be obtained by elution in a section where the proportion of mobile phase B was 40-45% in mobile phase A, with pH 7.0, containing 50 mM sodium phosphate and 2 M sodium chloride, and mobile phase B, with pH 7.0, containing 50 mM sodium phosphate and 30% acetonitrile, when a fusion protein having an IgG Fc domain purified by the purification method of the present invention is analyzed by hydrophobic interaction chromatography.

In an embodiment, the composition containing the fusion protein having an IgG Fc domain may contain the Peak 1 fraction in a content of 5% or less, or 0.001 % to 5%, but is not limited thereto.

In an embodiment, the Peak 1 fraction may include an amino acid sequence of at least one of SEQ ID NOS: 1 to 4.

In accordance with still another aspect of the present invention, there is provided a fusion protein purified by the purification method.

In an embodiment, the fusion protein may be aflibercept, but is not limited thereto.

### [Mode for Carrying Out the Invention]

Hereinafter, the present invention will be described in detail with reference to examples. However, these examples are merely preferable embodiments for illustrating the present invention, and are therefore not intended to limit the scope of the right of the present invention. Meanwhile, the technical matters not described herein can be sufficiently understood and easily implemented by a person skilled in the art or similar technical fields of the present application.

### [Example 1] Culture fluid harvesting and pretreatment

A culture fluid was prepared by culturing Chinese hamster ovarian (CHO) cells into which a vector containing a polynucleotide encoding, as a target protein, a fusion protein having an IgG Fc domain was introduced. A vector containing a polynucleotide encoding aflibercept as a representative example of the fusion protein was introduced into CHO cells, which were then cultured.

The culture fluid typically contains impurities including animal cells. Hence, before performing chromatography, the impurities including animal cells were removed by depth filtration. Through this procedure, a harvested cell culture fluid (HCCF) was prepared. The process speed and efficiency could be increased by adopting depth filtration to remove impurities and particles through a three-dimensional filter with a loose fiber structure and high porosity, unlike a surface filtration manner of removing impurities and particles through the filter material surface.

### [Example 2] Protein A affinity chromatography purification

For the direct product capture of a fusion protein (a fusion protein having an IgG Fc domain obtained by CHO cell culture, especially aflibercept) from the harvested cell culture fluid obtained through the pretreatment in Example 1, Protein A affinity chromatography was performed. This is a purification step using an affinity ligand that binds to an Fc domain of an antibody or an Fc-containing fusion protein, and proteins were eluted and recovered by using a buffer of pH 3-5, preferably pH 3-4, and more preferably about pH 3.5, for example, a citric acid and/or acetic acid buffer.

To this end, resin equilibration was performed by passing about 5 column volume (CV) of a tromethamine and/or sodium phosphate buffer of pH 6-8, preferably about pH 7, through a column at a flow rate with a retention time of 7.5 minutes. Thereafter, the harvested cell culture fluid obtained in Example 1 was injected into the equilibrated column, and the column was washed with 3 CV of an equilibration buffer to remove impurities adsorbed on the resin. Then, 10 CV of washing buffer 1 and 3-5 CV of washing buffer 2 were sequentially passed through the column to further remove impurities. A citric acid and/or acetic acid buffer of pH 5-6.5, preferably about pH 5.9, was used as washing buffer 1, wherein washing buffer 1 may further contain L-arginine and/or urea. A citric acid and/or acetic acid buffer of about pH 5.6 was used as washing buffer 2. Thereafter, 5 CV of a citric acid and/or acetic acid buffer of about pH 3.5 as an elution solution was passed through the column to recover a target protein. For the reuse of the resin, washing was sequentially performed with a strip buffer, 0.1 M sodium hydroxide, an equilibration buffer, and a storage buffer in this order after target protein recovery, and then the resin was stored.

In the present example, Protein A chromatography was performed in a binding and elution mode by using MabSelect SuRe resin.

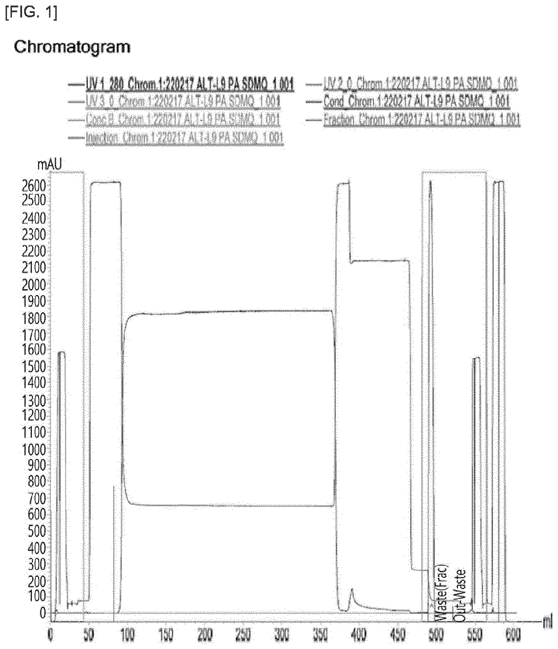

As a result, as shown in FIG. 1, the harvested cell culture fluid was purified by using Protein A affinity chromatography.

### [Example 3] Virus inactivation and neutralization

The leachate obtained by the Protein A affinity chromatography purification was titrated to pH 3.5 ± 0.1 and left for 1-2 hours, thereby inactivating potential viruses. After the inactivation reaction, the protein solution was neutralized under pH 5.5 ± 0.1 conditions, followed by microfiltration, thereby removing impurities such as aggregates.

### [Example 4] Depth filtration

Depth filtration is a step of mainly removing process-derived impurities by using a depth filter.

To this end, equilibration was performed by passing an equilibration buffer having 5 times the filter hold-up volume through a filter at a flow rate of 156 LMH (Liter/m²/h). As the elution buffer, a sodium phosphate and/or sodium acetate buffer of about pH 5.5 was used. After the equilibration, the protein solution was injected into the depth filter to recover a filtrate.

### [Example 5] Multimodal chromatography

Multimodal chromatography is a step of removing process-derived impurities, endotoxins, and potential viruses. The fusion protein having an IgG Fc domain, especially, aflibercept, obtained through CHO cell culture, was subjected to multimodal chromatography in a binding and elution mode using Capto Adhere resin.

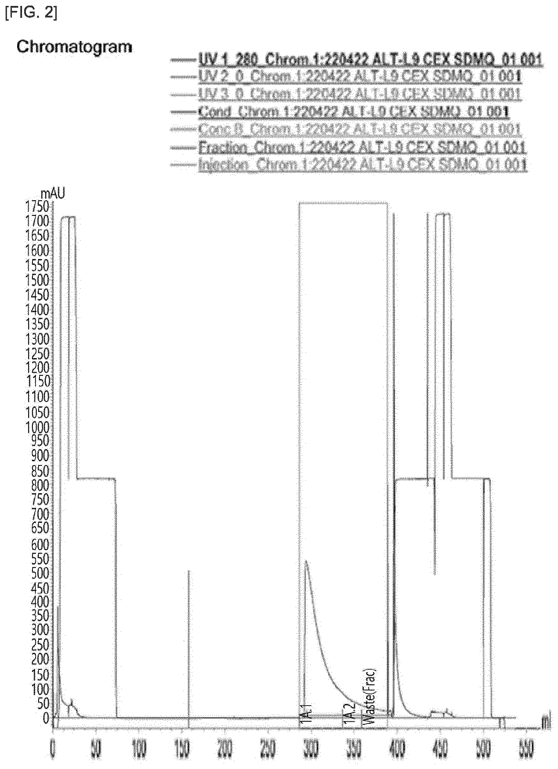

To this end, the resin was equilibrated by passing 5 CV of an equilibration buffer through a column at a flow rate of 150 cm/hr. A tromethamine and/or sodium phosphate buffer of about pH 7 was used as the equilibration buffer. Thereafter, the depth filtration recovery fluid obtained in Example 4 was injected and the column was washed with 5 CV of an equilibration buffer. A target protein was eluted and recovered by flowing 7 CV of an elution buffer through the column. As the elution buffer, a sodium phosphate and/or sodium acetate buffer of about pH 5 was used. For the reuse of the resin, washing was sequentially performed with a strip buffer, 1 M sodium hydroxide, an equilibration buffer, and a storage buffer in this order after protein recovery, and then the resin was stored. FIG. 2 shows the purification chromatogram results.

The virus log reduction results were obtained by performing multimodal chromatography. The results are shown in Table 5 in Example 7. A maximum log reduction of at least 6 was obtained for potential viruses, indicating an efficient virus removal effect.

### [Example 6] Cation exchange chromatography

Cation exchange chromatography is a final purification step for capturing only a fusion protein having an IgG Fc domain with an isoelectric point in a specific range.

To this end, resin equilibration was performed by passing 10 CV of an equilibration buffer at a rate of 160 cm/hr. As the equilibration buffer, a buffer of about pH 5 was used and, preferably, a sodium phosphate and/or sodium acetate buffer was used. The recovery fluid obtained by the multimodal chromatography step was injected, and the column was washed with 5 CV of an equilibration buffer. To remove highly acidic fractions among the target protein related substances, washing was performed by passing 3 CV of a sodium phosphate and/or sodium acetate buffer of about pH 5.9 through a column. As the elution buffer, 10 CV of a sodium phosphate and/or sodium acetate buffer of about pH 6.5 was used, thereby recovering the target protein. The protein recovery was performed at up to 8% of the maximum peak. For the reuse of the resin, washing was sequentially performed with a strip buffer, 1 M sodium hydroxide, an equilibration buffer, and a storage buffer in this order after protein recovery, and then the resin was stored.

Meanwhile, experiments based on the type of cation exchange resin chromatography and the corresponding purification conditions are shown in Table 1 below.

**TABLE 1**

| Resin | Condition | Binding condition | Washing condition | Eluting condition |
|---|---|---|---|---|
| Capto S | - | 50 mM NaPi | 50 mM NaPi | 50 mM NaPi |
| | | pH 5.0 | pH 5.9 | pH 6.5 |
| MegaCap II | pH adjusting | 50 mM NaPi | 50 mM NaPi | 50 mM NaPi |
| | | pH 4.5 | pH 5.0 | pH 6.0 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.1 | pH 6.1 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.0 | pH 6.0 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.0 | pH 5.8 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.0 | pH 6.1 |
| | | 50 mM NaCl | 50 mM NaCl | 50 mM NaCl |
| SP-550C | pH adjusting | 50 mM NaPi | 50 mM NaPi | 50 mM NaPi |
| | | pH 4.5 | pH 5.7 | pH 6.8 |
| | | 50 mM NaCl | 50 mM NaCl | 50 mM NaCl |
| SP-550C | pH adjusting | 50 mM NaPi | 50 mM NaPi | 50 mM NaPi |
| | | pH 4.5 | pH 5.9 | pH 6.8 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 6.0 | pH 6.5 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 6.0 | pH 6.6 |
| | | 50 mM NaCl | 50 mM NaCl | 50 mM NaCl |
| SP-650M | pH adjusting | 50 mM NaPi | 50 mM NaPi | 50 mM NaPi |
| | | pH 4.5 | pH 5.2 | pH 6.6 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.4 | pH 5.9 |
| | | 50 mM NaCl | 50 mM NaCl | 50 mM NaCl |
| | pH adjusting | 50 mM NaPi | 50 mM NaPi | 50 mM NaPi |
| | | pH 4.5 | pH 5.6 | pH 6.2 |
| | | 50 mM NaCl | 50 mM NaCl | 50 mM NaCl |
| Capto S ImpAct | 500 mg, 50 mg/mL (protein/resin) | 50 mM NaPi | 50 mM NaPi | 50 mM NaPi |
| | | pH 5.0 | pH 6.1 | pH 7.0 |
| | 200 mg, 20 mg/mL (protein/resin) | 50 mM NaPi | 50 mM NaPi | 50 mM NaPi |
| | | pH 5.0 | pH 6.1 | pH 7.0 |
| | 100 mg, 10 | 50 mM NaPi | 50 mM NaPi | 50 mM NaPi |
| | mg/mL (protein/resin) | pH 5.0 | pH 6.1 | pH 7.0 |

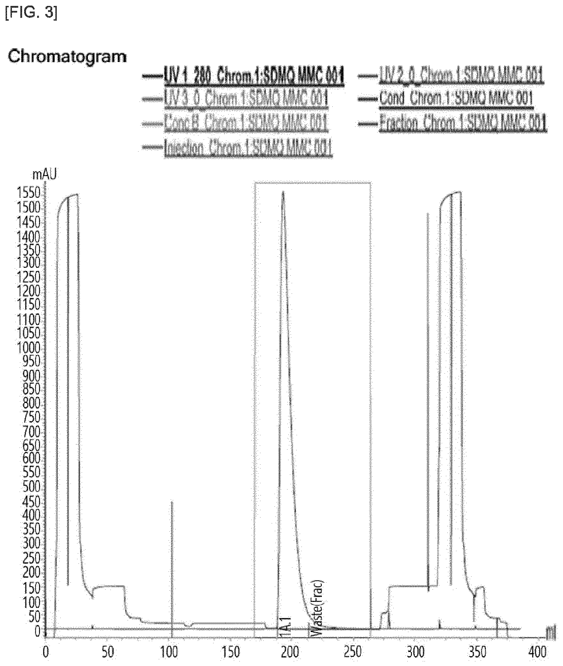

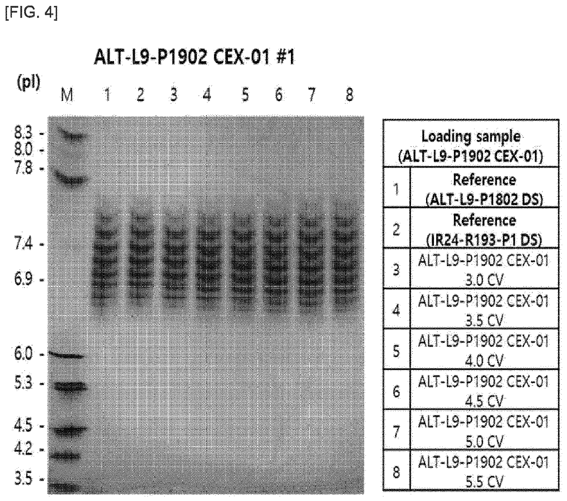

After the purification step (that is, Protein A affinity chromatography and multimodal chromatography) in Example 5, the fusion protein having an IgG Fc domain, especially, aflibercept, obtained from CHO cell culture, was subjected to cation exchange chromatography in a binding and elution mode by using Capto S resin, and the results are shown in FIG. 3. FIG. 4 shows the isoelectric focusing analysis results of fractions purified and eluted in the Capto S cation exchange chromatography purification step in FIG. 3. In FIG. 4, Lanes 1 and 2 represent internal references, and Lanes 3, 4, 5, 6, 7, and 8 represent analysis results according to the collection section of Capto S leachate. It can be seen that similar IEF patterns were observed despite an increase in the collection section.

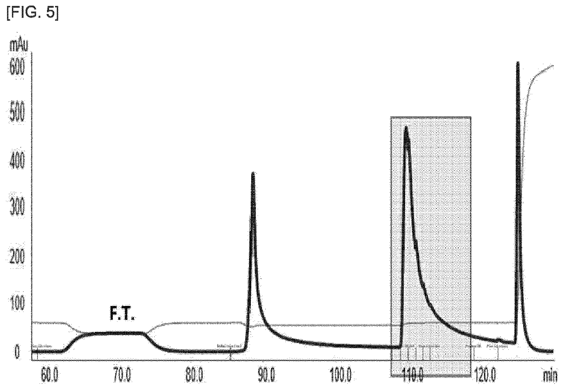

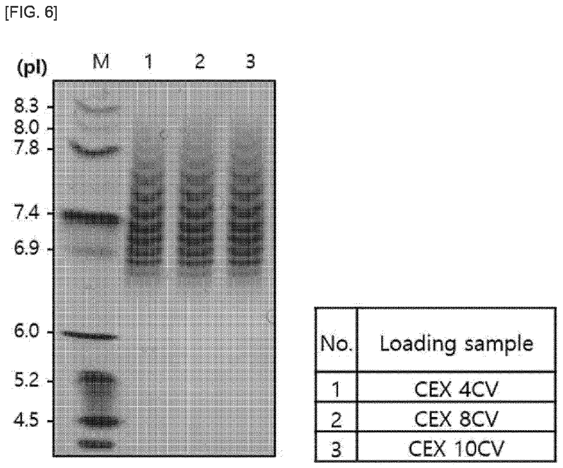

After the purification step (that is, Protein A affinity chromatography and multimodal chromatography) in Example 5, the fusion protein having an IgG Fc domain, especially, aflibercept, obtained from CHO cell culture, was subjected to cation exchange chromatography in a binding and elution mode by using SP-550C resin, and the results are shown in FIG. 5. FIG. 6 shows the isoelectric focusing analysis results of fractions purified and eluted in the SP-550C cation exchange chromatography purification step in FIG. 5.

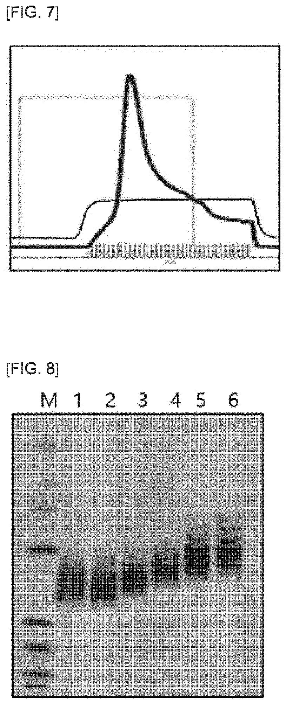

FIG. 7 shows a salt gradient elution chromatogram obtained by cation exchange chromatography. FIG. 8 shows the isoelectric focusing analysis results by fractions of salt gradient elution obtained by cation exchange chromatography. In the salt gradient elution, the yield of the target protein was reduced due to isoform removal.

### [Example 7] Evaluation of purification effect between multimodal chromatography and cation exchange chromatography

To evaluate the purification effect between multimodal chromatography and anion exchange chromatography, a comparison was made on the results of Capto adhere multimodal chromatography and Capto Q anion chromatography. To perform Capto adhere multimodal chromatography and Capto Q anion chromatography, AC, depth filtration 01, depth filtration 02, HIC, UF/DF, and depth filtration 03 were performed, and then the sample was dispensed and subjected to Capto adhere multimodal chromatography and Capto Q anion chromatography, respectively.

The same fusion protein samples containing an IgG Fc domain (especially, aflibercept; sample obtained from Examples 1 to 4) were purified by the Capto a chromatography and Capto adhere chromatography, respectively, and the residual host cell protein (HCP, a kind of impurity) of the eluted samples thus obtained was analyzed to compare the amounts of residual HCP in the respective samples. Capto Q anion exchange chromatography and Capto adhere multimodal chromatography were performed in the following manner.
AC chromatography
Depth filtration 01
Depth filtration 02
Hydrophobic Interaction Chromatography, *HIC*
UF/DF

### Capto Q anion exchange chromatography

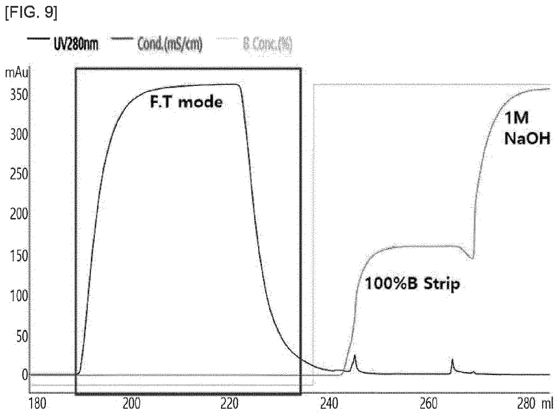

The Capto Q anion exchange chromatography was performed in a flow-through mode while Buffer A(Q) (50 mM sodium phosphate, pH 7.0) and Buffer B(Q) (50 mM sodium phosphate, 1 M sodium chloride, pH 7.0) were used as buffers. The results are shown in FIG. 9.

### Capto adhere multimodal chromatography

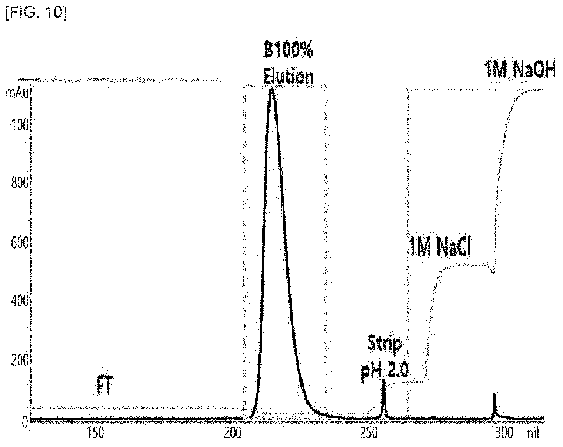

The Capto adhere multimodal chromatography was performed in a binding and elution mode under conditions where the same samples were loaded while Buffer A (Ad) (50 mM sodium phosphate, pH 7.0), Buffer B (Ad) (50 mM sodium phosphate, pH 5.0), and Buffer C (Ad) (20 mM sodium phosphate, 150 mM sodium chloride, pH 2.0) were used as buffers. The results are shown in FIG. 10.

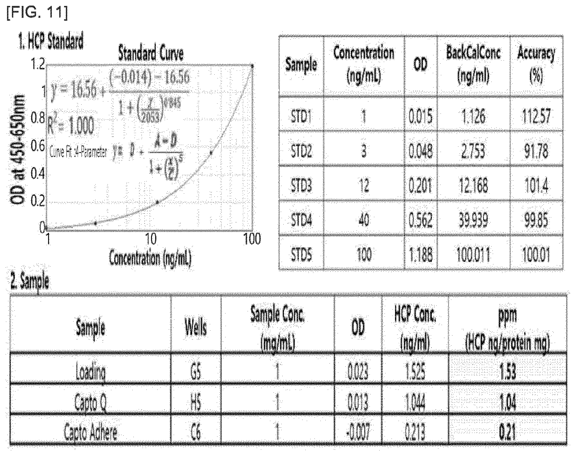

The residual host cell protein (HCP) of each of the samples eluted by Capto Q chromatography and Capto adhere chromatography was analyzed by using the Immunoenzymetric Assay for the Measurement of CHO Host Cell Protein kit (GYGNUS, F550). The analysis results are shown in FIG. 11. Table 2 below shows the residual HCP result values obtained in the respective purification steps including hydrophobic interaction chromatography (HIC) and anion exchange chromatography (AEX). Table 3 below shows the residual HCP result values obtained in the respective purification steps including multimodal chromatography. The HCP result values were determined by using a product from Cygnus, USA, by which the HCP in CHO cells was detected in an ELISA manner.

**TABLE 2**

| Purification step | HCP(ppm) |
|---|---|
| AC | 9471 |
| Depth filtration 1 | 320.9 |
| Depth filtration 2 | 44.6 |
| HIC | 21.5 |
| AEX | 6.7 |
| Formulation | 3.2 |

| | |
|---|---|
| * AC: Protein A chromatography, HIC: hydrophobic interaction chromatography, AEX: anion exchange chromatography | |

**TABLE 3**

| Purification step | HCP(ppm) |
|---|---|
| AC | 2420.6 |
| Depth filtration | 6.579 |
| UF | 6.542 |
| MMC | 5.319 |
| DS | <LOQ |

| | |
|---|---|
| * AC: Protein A chromatography, Depth filtration: depth filtration, UF: ultrafiltration, MMC: multimodal chromatography, AEX: anion exchange chromatography, DS: drug substance (purified stock solution), LOQ: limit of quantitation | |

As shown in Tables 2 and 3 above, the process (employing anion exchange chromatography) in Table 2 needed two runs of depth filtration to lower HCP, while the process (employing multimodal chromatography) in Table 3 needed only one run of depth filtration to lower HCP.

Table 4 below shows the contents of HCP, HCD, Protein A leachate and endotoxin according to each purification step. Table 5 below shows the amounts of residual viruses.

HCP was measured using a product from Cygnus, USA, by which the protein in CHO cells was detected in an ELISA manner, and HCD was measured using a product from Applied Biosystems, by which the DNA in CHO cells was detected in a real time - polymerase chain reaction (RT-PCR) manner. The protein A leachate was measured using a product from Repligen, by which the leakage content of protein A, a ligand for the resin, was detected in an ELISA manner. The content of endotoxin was measured using a product from Charles River, by which the content of endotoxin was detected by a method provided in the United States Pharmacopoeia.

LRV is obtained by measuring the residual amount of viruses after each process, by injecting a predetermined amount of viruses, in order to verify whether each process can remove the viruses, and a known method was used therefor.

In Table 5, Low pH Treatment corresponds to a process in Example 3; Capto Adhere corresponds to a process in Example 5; and Virus Filtration corresponds to a process performed after Example 6.

**TABLE 4**

| | HCP | | | HCD | | | Protein A leachate | | | Endotoxin | | |
|---|---|---|---|---|---|---|---|---|---|---|---|---|
| Step proce ss | Run 01 | Run 02 | Run 03 | Run 01 | Run 02 | Run 03 | Run 01 | Run 02 | Run 03 | Run 01 | Run 02 | Run 03 |
| | ppm | ppm | ppm | ng/do se | ng/do se | ng/do se | ppm | ppm | ppm | EU/ mg | EU/ mg | EU/ mg |
| HCCF | 958, 391 | 826, 951 | 1,161, 068 | 431.5 738 | 796.2 512 | 662.2 885 | | | | | | |
| PA | 4,40 8 | 2,65 5 | 4,922 | 0.175 4 | 0.018 3 | 0.066 6 | 10.71 2 | 2.592 | 0.632 | 0.0 278 | 0.0 250 | 0.0 238 |
| Depth filtrati on | 6 | 11 | 13 | <0.00 20 | <0.00 18 | <0.00 18 | <0.4( ND) | <0.4( ND) | <0.4( ND) | 0.0 333 | 0.0 294 | 0.0 294 |
| UF-01 | 9 | 15 | 17 | <0.00 03 | <0.00 02 | <0.00 03 | <0.4( ND) | <0.4( ND) | <0.4( ND) | 0.0 064 | 0.0 040 | 0.0 042 |
| MMC | n.a. | 9 | 9 | n.a. | <0.00 16 | <0.00 15 | n.a. | <0.4( ND) | <0.4( ND) | 0.0 278 | 0.0 263 | 0.0 250 |
| CEX | <1 | <1 | <1 | <0.00 38 | <0.00 30 | <0.00 60 | <0.4( ND) | <0.4( ND) | <0.4( ND) | 0.0 625 | 0.0 500 | 0.1 000 |
| Virus filtrati on | <1 | <1 | <1 | <0.00 43 | <0.00 43 | <0.00 75 | <0.4( ND) | <0.4( ND) | <0.4( ND) | 0.0 714 | 0.0 625 | 0.1 25 |
| UF/D F | <1 | <1 | <1 | <0.00 01 | <0.00 00 | <0.00 00 | <0.4( ND) | <0.4( ND) | <0.4( ND) | 0.0 011 | 0.0 070 | 0.0 008 |
| Drug-subst ance | <1 | <1 | <1 | <0.00 01 | 0.000 1 | <0.00 01 | <0.4( ND) | <0.4( ND) | <0.4( ND) | 0.0 011 | 0.0 043 | 0.0 010 |

| | | | | | | | | | | | | |
|---|---|---|---|---|---|---|---|---|---|---|---|---|
| * HCCF: harvested cell culture fluid, PA: Protein A chromatography, Depth filtration: depth filtration, UF: ultrafiltration, MMC: multimodal chromatography, CEX: cation exchange chromatography, UF/DF: ultrafiltration/diafiltration, HCP: host cell protein, HCD: host cell DNA, Protein A leachate: Protein A leachate, Endotoxin: endotoxin | | | | | | | | | | | | |

**TABLE 5**

| Virus Removal | Minimum LRV | | | |
|---|---|---|---|---|
| | MVM | MuLV | Reo-3 | PRV |
| Low pH Treatment | N/A | ≥ 6.44 | N/A | ≥ 8.05 |
| Capto Adhere | 3.31 | ≥ 5.44 | ≥ 6.27 | ≥ 6.16 |
| Virus Filtration | ≥ 7.79 | ≥ 5.84 | ≥ 5.88 | ≥ 6.30 |

| | | | | |
|---|---|---|---|---|
| LRV: log-reduction value, Minimum LRV: minimum light reflectance value, MVM: minute virus of mice, MuLV: murine leukemia virus, Reo-3: Reovirus type 3, PrV: pseudorabies virus | | | | |

### [Example 8] Analysis of oxidation/deamidation/dehydration of purified aflibercept

The aflibercept sample (Aflibercept 1) purified by the method of the present invention and a commercially available aflibercept sample (Aflibercept 2) were analyzed for oxidation, deamidation, and dehydration.

The purified aflibercept sample (1 mg) obtained from Examples 1 to 6 was mixed with 10 µl of 1 M dithiothreitol, and 1 ml of a denaturing buffer (7 M Guanidine hydrochloride, 0.25 M Tris-HCI, pH 8.4) was prepared. Then, the protein sample was reduced by incubation at 37°C for 1 hour. Thereafter, 10 µl of 1 M iodoacetamide was added to the reduced solution to alkylate the solution by reacting for 1 hour. The buffer solution was substituted with 50 mM Tris-HCI (pH 7.8) by using a centrifugal filter tube, and 50 µl of the sample thus prepared and 2 µl of 1 mg/mL trypsin were mixed, followed by reaction at 37°C for 18 hours, thereby breaking down the protein into peptides. The obtained peptide samples of each sample were subjected to LC-MS analysis and LC-MS/MS analysis by reverse-phase ultraperformance chromatography (RP-UPLC, ThermoFisher, USA) and online electrospray ionization mass spectrometry absorption (Q-TOF, ThermoFisher, USA), separately, and the results were analyzed using Byonic software (Protein Metrics, USA).

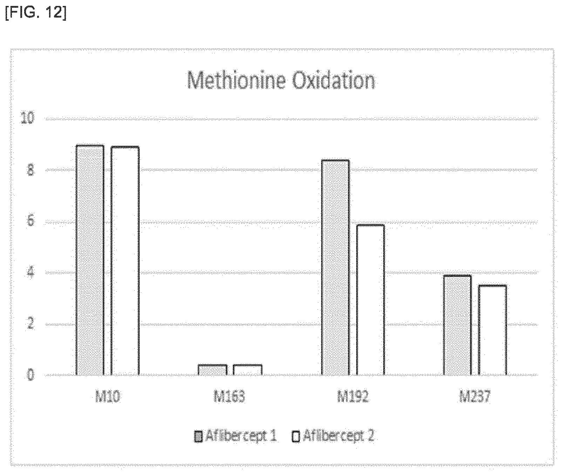

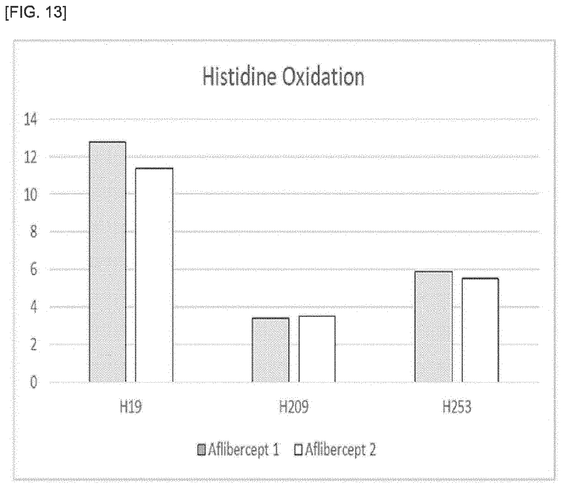

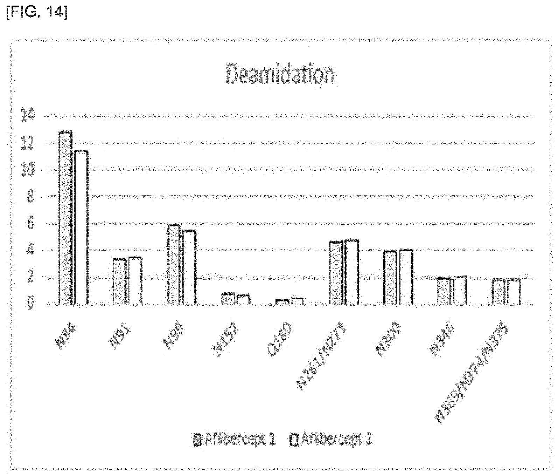

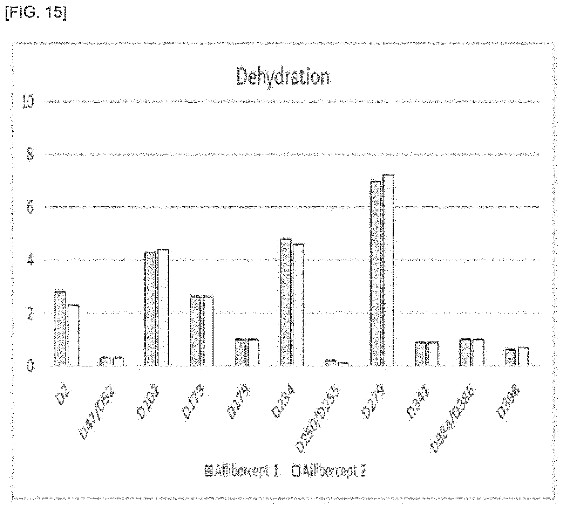

The oxidation analysis results were obtained according to the type of oxidized amino acid, and the oxidation analysis results of methionine residues (10th, 163rd, 192nd, and 237th amino acids) are shown in FIG. 12, and the oxidation analysis results of histidine residues (19th, 209th, and 253rd amino acids) are shown in FIG. 13. The deamidation and dehydration analysis results are shown in FIGS. 14 and 15, respectively (84th, 91st, 99th, 152nd, 180th, 261st, 271st, 300th, 346th, 369th, 374th, and 375th amino acids; and 2nd, 47th, 52nd, 102nd, 173rd, 179th, 234th, 250th, 255th, 279th, 341st, 384th, 386th, and 398th amino acids).

As shown in FIGS. 12 and 13, oxidation primarily occurs in the methionine residues and was observed in some histidine residues but not in tryptophan residues.

The methionine oxidation sites were observed in four amino acid residues, Methionine #10 (M10), Methionine #163 (M163), Methionine #192 (M192), and Methionine #237 (M237). All the degrees of oxidation were 10% or less, and similar results were obtained in the sample purified by the method of the present invention (Aflibercept 1) and the commercially available sample (Aflibercept 2).

The histidine oxidation sites were observed in three amino acid residues, Histidine #19 (H19), Histidine #209 (H209), and Histidine #253 (H253). All the degrees of oxidation were 0.5% or less, and similar results were obtained in the sample purified by the method of the present invention (Aflibercept 1) and the commercially available sample (Aflibercept 2).

As shown in FIG. 14, deamidation was observed in some asparagine and glutamine residues. The deamidation sites were observed in nine amino acid residues, Asparagine #84 (N84), Asparagine #91 (N91), Asparagine #99 (N99), Asparagine #152 (N152), Glutamine #180 (Q180), Asparagine #261/#271 (N261/N271), Asparagine #300 (N300), Asparagine #346 (N346), and Asparagine #369/#374/#753 (N369/N374/N753). With respect to N261/N271 and N369/N374/N753, the occurrence of deamidation was confirmed in any one of the asparagine residues at the indicated positions, but the analysis to identify the exact residues was not conducted. All the degrees of deamidation were 10% or less, with the exceptions being 12.8% and 11.4% in the degree of deamidation of N84. Similar results were obtained in the sample purified by the method of the present invention (Aflibercept 1) and the commercially available sample (Aflibercept 2).

As shown in FIG. 15, dehydration was observed in some aspartate residues. The dehydration sites were observed in 11 amino acid residues including Aspartate #2 (D2), Aspartate #39 (D39), Aspartate #47/#52 (D47/D52), Aspartate #102 (D102), Aspartate #173 (D173), Aspartate #179 (D179), Aspartate #234 (D234), Aspartate #250/#255 (D250/D255), Aspartate #297 (D297), Aspartate #341 (D341), and Aspartate #384/#386 (D384/386). All the degrees of dehydration were 10% or less, and similar results were obtained in the sample purified by the method of the present invention (Aflibercept 1) and the commercially available sample (Aflibercept 2).

The above results suggest that the purification method of the present invention can effectively purify aflibercept because both the sample purified by the method of the present invention (Aflibercept 1) and the commercially available sample (Aflibercept 2) showed similar trends.

### [Example 9] Analysis of hydrophobic interaction chromatography of purified aflibercept and each fraction thereof

The sample purified by the method of the present invention (Aflibercept 1) and the commercially available sample (Aflibercept 2) were analyzed by hydrophobic interaction chromatography. The composition of mobile phase A was 50 mM sodium phosphate and 2 M sodium chloride, with pH 7.0, and the composition of mobile phase B was 50 mM sodium phosphate and 30% acetonitrile, with pH 7.0. The analysis column was TSKGel^{®} Phenyl-5PW (TOSOH, Cat No. 0007573), and the analysis conditions were as follows: elution was performed by increasing the proportion of mobile phase B from 0% to 90% for 15 minutes with a column oven temperature of 30°C and a flow rate of 1 mL/min. In this analysis, the aflibercept sample purified by the method of the present invention (Aflibercept 1) and the commercially available aflibercept sample (Aflibercept 2) were used. According to the proportion of each hydrophobic interaction chromatography fraction of purified aflibercept shown in Table 6, similar results were obtained in the two aflibercept samples. Peak 1 was eluted in the section with 40-45% of mobile phase B, Peak 2 was eluted in the section with 50-55% of mobile phase B, and Peak 3 was eluted in the section with 70-80% of mobile phase B.

**TABLE 6**

| | Aflibercept 1 | Aflibercept 2 |
|---|---|---|
| Peak 1 | 0.88% | 1.76% |
| Peak 2 | 91.86% | 90.58% |
| Peak 3 | 7.26% | 7.66% |

The proteins of the Peak 1 and Peak 2 fractions observed in the HI-HPLC analysis were subjected to intact mass analysis of reduced and deglycosylated forms by using LC-MS. As a result of the intact mass analysis of the Peak 1 fraction, protein fragments with the amino acid sequences shown in the peptide fragment analysis results of hydrophobic interaction chromatography Peak 1 fraction of the purified aflibercept as shown in Table 7 below were analyzed. Out of these, the 202-431 peptide was analyzed as a major form (peak 1-d), which was assumed to be a form in which VEGFR1 domain 2 and VEGFR2 domain 3 were mostly deleted. The Peak 2 fraction was sub-divided into Peak 2-1, Peak 2-2, and Peak 2-3, and no difference was observed in the intact mass analysis results among the fractions.

**TABLE 7**

| | M.W | Seq. | | | Theoretical (Da) | |
|---|---|---|---|---|---|---|
| Peak1-a | 24934.0 | 210 | 431 | - | 24938.3 | -4.3 |
| Peak1-b | 25172.0 | 208 | 431 | +TH | 25176.5 | -4.5 |
| Peak1-c | 25810.0 | 203 | 431 | +HEKDKTH | 25814.3 | -4.3 |
| **Peak1-d** | **25909.5** | **202** | **431** | **+VHEKDKTH** | 25913.4 | -3.9 |

The proteins of Peak 1 and Peak 2 fractions observed in the HI-HPLC analysis were subjected to post-translational modification analysis using LC-MS. The deamidation analysis results of hydrophobic interaction chromatography fractions of purified aflibercept shown in Table 8 below indicated that the deamidation of asparagine residues was similar between Peak 1 fraction and Peak 2 fraction, with no difference.

**TABLE 8**

| | **Peak 1** | **Peak 2-1** | **Peak 2-2** | **Peak 2-3** |
|---|---|---|---|---|
| N68 | 1% | 1% | 1% | 1% |
| N84 | 28% | 27% | 27% | 28% |
| N99 | 4% | 5% | 2% | 2% |
| N271 | 1% | 1% | 1% | 1% |
| N300 | 10% | 9% | 9% | 10% |
| N369/N374/N375 | 4% | 4% | 4% | 4% |

The methionine oxidation analysis results of the hydrophobic interaction chromatography fractions of the purified aflibercept shown in Table 9 confirmed that the oxidation of methionine residues in the protein of the Peak 1 fraction was approximately 2-3 times those of the proteins of the Peak 2 fraction. Both of M10/M20 of T1 peptide and M237 of T28 peptide were increasingly oxidized.

**TABLE 9**

| | **Peak 1** | **Peak 2-1** | **Peak 2-2** | **Peak 2-3** |
|---|---|---|---|---|
| M10/M20 | **7%** | 4% | 2% | 2% |
| M237 | **10%** | 6% | 3% | 3% |

### [Example 10] Hydrophobic interaction chromatography of purified aflibercept and Anti-VEGF bioassay of each fraction

To evaluate the activities of proteins of the Peak 1 and Peak 2 fractions observed in the HI-HPLC assay, Pathhunter anti-VEGF bioassay (Eurofins Discoverx, USA) was performed. PathHunter cells responding to VEGF were mixed with each hydrophobic interaction chromatography fraction of the purified aflibercept in culture fluids, and the activity of each fraction was measured by investigating the effect on VEGF binding through the comparison with the reference.

As for the Peak 1 fraction, the relative titer was analyzed to be 6.6%, indicating that the activity was reduced due to the deletion of the VEGFR domain. As for the Peak 2 fraction, the relative titers were analyzed to be maintained, indicating no reduction in activity.

While the present invention has been described with reference to the particular illustrative embodiments, a person skilled in the art to which the present invention pertains can understand that the present invention may be embodied in other specific forms without departing from the technical spirit or essential characteristics thereof. Therefore, the embodiments described above should be construed as being exemplified and not limiting the present invention. The scope of the invention should be construed as the meaning and scope of the appended claims rather than the detailed description and all changes or variations derived from the equivalent concepts fall within the scope of the present invention.

## Claims

1. A method for purifying a fusion protein having an IgG Fc domain, wherein a raw material containing a fusion protein having an IgG Fc domain is subjected to a multimodal chromatography purification step to obtain a fusion protein having an IgG Fc domain, with endotoxin < 0.05 EU/mg or a size exclusion HPLC purity of 98% or more.

2. The method of claim 1, wherein a cation exchange chromatography purification step is added.

3. The method of claim 1 or 2, wherein no size exclusion chromatography, hydrophobic interaction chromatography, and anion exchange resin chromatography are performed.

4. The method of claim 1, wherein a ligand for the multimodal chromatography includes a substance having multimodal functions of ionic binding, hydrogen binding, and hydrophobic binding.

5. The method of claim 2, wherein a ligand of the cation exchange chromatography includes at least one of sulfonic acid (-SOs), sulfopropyl, and carboxymethyl.

6. The method of claim 1 or 2, wherein in the chromatography purification step, elution is performed using a pH gradient.

7. The method of claim 1 or 2, wherein the chromatography includes an elution stage, and the elution stage is performed in a binding and elution mode.

8. The method of claim 1 or 2, wherein the fusion protein having an IgG Fc domain is a vascular endothelial cell growth factor antagonist.

9. The method of claim 1 or 2, wherein the fusion protein having an IgG Fc domain is aflibercept.

10. A method for purifying a fusion protein having an IgG Fc domain, the method comprising:
a) culturing cells producing a fusion protein having an IgG Fc domain;
b) recovering a protein from the cells cultured in step a);
c) purifying the protein, recovered in step b), by primary chromatography;
d) purifying the protein, purified by primary chromatography in step c), by multimodal chromatography and cation exchange chromatography,
wherein no anion exchange chromatography is used.

11. The method of claim 10, wherein the fusion protein having an IgG Fc domain, finally purified by the method, has a size exclusion HPLC purity of 98% or more, endotoxin < 0.05 EU/mg, or a pl range of 6.0-8.0.

12. The method of claim 10, wherein the fusion protein having an IgG Fc domain is a vascular endothelial cell growth factor antagonist.

13. The method of claim 10, wherein the fusion protein having an IgG Fc domain is aflibercept.

14. The method of claim 10, wherein the purifying by the multimodal chromatography in step d) comprises:
(a) loading the protein, purified by primary chromatography, onto a multimodal chromatography column equilibrated with a buffer of pH range of 6-8; and
(b) injecting a buffer having a pH lower than that in step (a) into the chromatography column to elute and harvest the purified protein under conditions of pH range of 4-6.

15. The method of claim 10, wherein a ligand for the multimodal chromatography includes a substance having multimodal functions of ionic binding, hydrogen binding, and hydrophobic binding.

16. The method of claim 10, wherein the purifying by the cation exchange chromatography in step d) comprises:
(a) loading the protein, purified by the preceding chromatography, onto a cation exchange chromatography column equilibrated with a buffer of pH range of 4-6; and
(b) harvesting a fusion protein having an IgG Fc domain with a pl range of 6.0-8.0 by elution with a buffer having a pH higher than that of the buffer in step (a).

17. The method of claim 16, wherein in step (b), the buffer having a pH higher than that of the buffer in step (a) has a pH range of 5-8.

18. The method of claim 10, wherein a ligand of the cation exchange chromatography includes at least one of sulfonic acid (-SOs), sulfopropyl, and carboxymethyl.

19. The method of claim 10, wherein at least one of the multimodal chromatography and the cation exchange chromatography includes an elution stage, and the elution stage is performed in a binding and elution mode.

20. The method of claim 10, wherein no size exclusion chromatography and hydrophobic interaction chromatography are performed.

21. The method of claim 10, wherein before, after, or a combination of before and after at least one of steps c) and d), at least one of a depth filtration step, an ultrafiltration step, a dialysis filtration step, and a virus inactivation step is additionally performed.

22. The method of claim 10, wherein at least one step selected from the group consisting of c) purifying by primary chromatography and d) purifying by multimodal chromatography and cation exchange chromatography includes an elution stage, and the elution stage uses a pH gradient.

23. A composition comprising a fusion protein having an IgG Fc domain purified by the method of claims 1 or 10.

24. The composition of claim 23, wherein the composition comprises a Peak 1 fraction obtained by elution in the section with 40-45% of mobile phase B, as analyzed by hydrophobic interaction chromatography using mobile phase A with 50 mM sodium phosphate, 2 M sodium chloride, and pH 7.0 and mobile phase B with 50 mM sodium phosphate, 30% acetonitrile, and pH 7.0.

25. The composition of claim 24, wherein the composition comprises 5% or less of the Peak 1 fraction.
